# EUROPEAN PATENT SPECIFICATION

(11) **EP 1 285 961 B1**
(45) Date of publication and mention of the grant of the patent: **14.11.2007**
(21) Application number: 01917709.6
(22) Date of filing: 30.03.2001
(51) Int. Cl.: C12N 5/06, C12Q 1/02

(54) **METHOD OF FORMING VERTEBRATE PANCREAS IN VITRO**
VERFAHREN ZUR SCHAFFUNG VON WIRBELTIER-SPEZIFISCHER BAUCHSPEICHELDRÜSE IN VITRO
PROCEDE DE FORMATION IN VITRO DE PANCREAS DE VERTEBRE

(30) Priority: 31.05.2000 JP 2000161795
(43) Date of publication of application: 26.02.2003
(73) Proprietor: Japan Science and Technology Agency, Kawaguchi-shi Saitama (JP)
(72) Inventor: ASASHIMA, Makoto, Tokyo 170-0005 (JP); MORIYA, Naomi, Mitaka-shi, Tokyo 181-0001 (JP)
(74) Representative: Williams, Aylsa
(86) International application number: PCT/JP2001/002765
(87) International publication number: WO 2001/092480

(56) References cited:
- NAOMI MORIYA ET AL.: 'In vitro organogenesis of pancreas in Xenopus laevis dorsal lips treated with retinoic acid' DEVELOPMENT GROWTH & DIFFERENTIATION vol. 42, no. 2, April 2000, pages 175 - 185, XP002941460
- HIROMASA NINOMIYA ET AL.: 'Endoderm differentiation and inductive effect of activin-treated ectoderm in Xenopus' DEVELOPMENT GROWTH & DIFFERENTIATION vol. 41, no. 4, 1999, pages 391 - 400, XP002941461
- NAOMI MORIYA ET AL.: 'In vitro pancreas formation from Xenopus ectoderm treated with activin and retinoic acid' DEVELOPMENT GROWTH & DIFFERENTIATION vol. 42, no. 6, December 2000, pages 593 - 602, XP002941462
- NAOMI MORIYA ET AL.: 'Induction of pronephric tubules by activin and retionic acid in presumptive ectoderm of Xenopus laevis' DEVELOPMENT GROWTH & DIFFERENTIATION vol. 35, no. 2, 1993, pages 123 - 128, XP002941463

## Description

### Technical Field

The present invention relates to a method of forming pancreas in vitro, more particularly, a method of forming pancreas in vitro wherein a presumptive ectodermal region of a vertebrate blastula is treated with activin and retinoic acid with time lag and then cultured, and a screening method of a substance effective for the diagnosis and treatment of diseases attributed to pancreas induced in vitro and pancreas using the pancreas induced in vitro.

### Background Art

Every multicellular organism starts its development by fertilization and is completed as an individual having various tissues and well-balanced system by undergoing cell division (cleavage) and cell differentiation. The differentiation process is highly complicated and is thought that important interactions between cells called induction phenomena takes place in many steps of the differentiation stratum. The elucidation of "molecule that dominates morphogenesis" is said to be the most significant. Amphibian embryos are often and mostly used as materials for these studies, nevertheless, the basic rule of body formation is common to all the vertebrates and homologous genes are known to have quite a similar function even among different species.

Amphibian embryo has conventionally been regarded as an extremely valuable material in the field of experimental embryology with which many studies have been made. This is because amphibian egg fertilizes and develops externally, its large egg makes embryo operation possible, and its time course changes can easily be observed. The amphibian blastopore upper lip of gastrula is a special region and when it is transplanted into the ventral side of another embryo, a secondary embryo including head or body-tail part is induced. This is why the blastopore upper lip is named "organizer" as a region that acts as the center of morphogenesis that determines the embryo system. It is well known that the organizer induces central nerve by functioning to presumptive ectoderm during invagination of the primitive gut, while the organizer itself differentiates into dorsal mesoderm and anterior endoderm.

On the other hand, pancreas is an endocrine organ that indicates histomorphology and manner of development common to most vertebrates, namely, mammals, birds, reptiles and amphibians, and is an exocrine organ as well. It is known that during the developmental process, dorsal and ventral primordia arise from the endoderm, and they fuse to form pancreas (Development 121, 1569-1580, 1995).

It has been said that there exists a mesoderm in the vicinity of endoderm in the process of embryogenesis, and that action from mesenchyme to endoderm is necessary for the differentiation of pancreas (Dev. Biol. 4, 242-255, 1962). Recent studies have reported that involvement of notochord is required for pancreatic formation of chick, and that notochord suppresses the Shh expression in the endoderm in its vicinity to differentiate pancreas. It has also been reported that it is the endoderm of the pancreas presumptive region that differentiates into pancreas by action of notochord, and that differentiation into pancreas is not found in endoderm aside from the pancreas presumptive region, even when notochord coexists (Development 124, 4243-4252, 1997, Proc. Natl. Acad. Sci. USA 95, 13036-13041, 1998, Genes and Dev. 12, 1705-1713, 1998).

Further, from research at gene level, it had been reported that homeobox gene, known as ipf-1 and pdx-1 that express in the pancreatic primordium of mouse, is essential to the formation process of pancreas. Gene targeting experiment of ipf-1 revealed that mouse embryo without this gene was pancreas-defective (Nature 371, 606-609, 1994). However, the primordium of pancreas was formed even when this gene was deficient, and the existence of glucagon-positive cells was confirmed (Development 122, 983-995, 1996). In addition, it is known that the vegetal pole cell of Xenopus blastula expresses both of XlHbox8 that is a pancreas-specific transcription factor and the homolog of PDX-1 and IFABP that is a small intestinal epithelium marker, however, when the signal of TGF-β system at the endoderm is inhibited, the expression of XlHbox8 is inhibited (Development 122, 1007-1015, 1996).

On the other hand, it is known that retinoic acid is a regulatory factor for the embryonic patterning along the anteroposterior axis (Nature 340, 140-144, 1989, Development 112, 945-958, 1991, Dev. Biol. 192, 1-16, 1997, Zool. Sci. 15, 879-886, 1998), and that this retinoic acid transforms anterior neural tissue of Xenopus embryo to a posterior one and is effective on mesodermal development (Genes Dev. 5, 175-187, 1991, Develop. Growth. Differ. 35, 123-128, 1993). It has also been reported that treatment with activin induces most mesodermal tissues such as notochord, muscle, mesenchyme and coelomic epithelium, dose-dependently in Xenopus animal cap cells (Roux's Arch. Dev. Biol. 198, 330-335, 1990, Nature 347, 391-394, 1990, Roux's Arch. Dev. Biol. 200, 230-233, 1991). Changing the dosage of retinoic acid that is co-treated with activin enables the mesodermal tissues such as notochord, muscle and pronephros that differentiate from animal cap cells to be lateralized and posteriorized (Develop. Growth. Differ. 35, 123-128, 1993).

As to the action of retinoic acid to the endodermal organ, it has been reported by Dixon et al. that when Xenopus embryos at developmental stage 22 to 32 are treated with retinoic acid, the morphology of the digestive organs such as intestines, liver and stomach become abnormal, however, it has also been reported that pancreas of Xenopus embryos at developmental stage 22 to 32 that had been treated with retinoic acid is formed normally, and no effect is found in the expression of XlHbox8, an endoderm-specific marker (Dev. Genes Evol. 208, 318-326, 1998).

Heretofore, specific induction of a specific organ in vitro had been regarded as being extremely difficult, and although pancreas is an endocrine and exocrine organ that plays an important role in vivo, the complex differentiation and formation mechanisms of pancreas remain unclear. The present inventors have reported that treating the blastopore upper lip cell of Xenopus early gastrula with retinoic acid enables pancreas formation with high efficiency (Moriya, N. et al.; Develop. Growth. Differ. 42, 175-185, 2000). However, this system uses blastopore upper lip cell primarily having an autonomous differentiation ability whose test system is still complex to elucidate the mechanism of pancreas differentiation, although this system can form pancreas with high efficiency. The object of the present invention is to provide a method, whereby a pancreas induced in vitro which enables to obtain findings on the differentiation and formation mechanisms of pancreas and thus is useful in developmental engineering or organ engineering, a pancreas for transplantation by which it can be evaluated whether or not a pancreas induced in vitro can function in practice in vivo, and a pancreas induced in vitro which contributes to the development of diagnosis and treatment of pancreatic diseases of higher animals, can be artificially and efficiently induced from a gastrula excluding the presumptive region of pancreas.

### Disclosure of the Invention

The present inventors have conducted intensive study to elucidate the object mentioned above, and have discovered that a morphological and functional pancreas can be formed with high efficiency in vitro in the following manner. A presumptive ectodermal region of undifferentiated cells of late blastula, which, by nature, forms irregular epidermis but not pancreas when cultured in vitro, was cut out from late blastula of Xenopus, treated with activin, and then treated with retinoic acid after three to five hours. These explants were subjected to stationary culture in Steinberg's solution containing BSA to thereby convert the fate toward pancreas. The present invention had been accomplished based upon this finding.

The present invention relates to: a method of forming vertebrate pancreas in vitro, wherein a piece of presumptive ectoderm of a vertebrate blastula or gastrula is treated with activin and retinoic acid in vitro, then cultured wherein treatment with activin is conducted, followed by treatment with retinoic acid after a predetermined time lag and wherein said vertebrate blastula or gastrula is non-human (claim 1); the method of forming vertebrate pancreas in vitro according to claim 1, wherein treatment with activin is conducted, followed by treatment with retinoic acid after three to 15 hours (claim 2); the method of forming vertebrate pancreas in vitro according to any of claims 1 to 2, wherein the treatment with activin is a stationary culture treatment with activin at a concentration of 50 to 150 ng/ml for 0.5 to two hours (claim 3); the method of forming vertebrate pancreas in vitro according to any of claims 1 to 3, wherein the treatment with retinoic acid is a stationary culture treatment with retinoic acid at a concentration of more than 10⁻⁵ M for 0.5 to two hours (claim 4); the method of forming vertebrate pancreas in vitro according to any of claims 1 to 4, wherein the culture thereafter is a stationary culture treatment in a physiological saline (claim 5); the method of forming vertebrate pancreas in vitro according to any of claims 1 to 5, wherein the vertebrate is an animal that belongs to amphibian (claim 6); and the method of forming vertebrate pancreas in vitro according to claim 6, wherein the animal that belongs to amphibian is a Xenopus (claim 7).

The present invention further relates to a screening method for a substance capable of curing hypofunction or dysfunction of pancreas wherein the pancreas is obtained by the method of forming vertebrate pancreas in vitro according to any one of claims 1 to 7 (claim 8).

### Brief Description of Drawings

Figure 1 is a view showing the method of treating a piece of presumptive ectoderm of a Xenopus embryo with activin and retinoic acid.
Figure 2 is a view showing the image observed by light microscopy of tissues that differentiate in the explants by treatment of various kinds.
Figure 3 is a view showing the image observed by light microscopy of explants treated with activin and retinoic acid.
Figure 4 is a view showing the expression patterns of pancreas-specific genes.
Figure 5 is a view showing the immunohistochemistry of explants treated with activin and retinoic acid.

### Best Mode of Carrying Out the Invention

There is no particular limitation to the method of forming vertebrate pancreas in vitro in the present invention, as long as it is a method wherein a piece of presumptive ectoderm that is an undifferentiated cell of vertebrate blastula is treated with activin and retinoic acid in vitro, then cultured, wherein treatment with activin is conducted, followed by treatment with retinoic acid after a predetermined time lag and wherein said vertebrate blastula or gastrula is non-human, thereby enabling the differentiation and induction of pancreas in vitro. Aside from the pancreas organ induced in vitro, the pancreas in the method of forming pancreas in vitro of the present invention includes the following, for convenience: an explant having an expression ability of a pancreas-specific molecular marker gene, such as insulin gene, homeobox gene such as IPF1, PDX1 or the like, XIHbox8 gene that is a pancreas-specific transcription factor and the homolog of PDX1; an explant having a cytomorphology that is similar to pancreas in vivo; and an explant having a secretory gland-like structure that is similar to pancreas in vivo.

There is no particular limitation to the vertebrate mentioned above, as long as it is a vertebrate having a pancreas that belongs to non-human mammal, bird, reptile and amphibian. Nevertheless, in a level where findings on the differentiation and formation mechanisms of pancreas that are useful in developmental engineering or organ engineering can be obtained, an animal that belongs to amphibian, which is relatively easy to handle and abundant knowledge of which developmental engineering or organ engineering has been obtained to the present, a Xenopus, specifically, can particularly be exemplified as a preferable vertebrate. The fact that pancreas could be induced in vitro by the use of undifferentiated cells of Xenopus, which is a vertebrate, suggests that pancreas can be induced in vitro by using ES cells that are undifferentiated cells of mammals, including human.

As the blastula mentioned above, a mid-blastula to late-blastula can preferably be used, and a specific example of said mid-blastula to late-blastula is that of a Xenopus at developmental stage 8 to 10. The developmental stage of this Xenopus can be determined from the criterion as described previously (Nieuwkoop, P.D., Faber, J., 1956. Normal Table of Xenopus laevis. North-Holland Pub. Co. Amsterdam.).

As the method of treatment with activin and retinoic acid mentioned above, there is no particular limitation as long as it is a method of treatment wherein a presumptive ectoderm that is an undifferentiated cell of vertebrate blastula is treated with activin and retinoic acid in vitro, then cultured, wherein treatment with activin is conducted, followed by treatment with retinoic acid after a predetermined time lag and wherein said vertebrate blastula or gastrula is non-human, thereby enabling the differentiation and induction of pancreas. In the treatment with activin followed by treatment with retinoic acid, after a predetermined time, the predetermined time is preferably three to 15 hours later, more preferably three to five hours later. Treatment with activin followed by treatment with retinoic acid after a time lag of three to 15 hours, specifically three to five hours, enables the pancreas to be induced at a much higher rate. During the time lag, it is preferable for the piece of presumptive ectoderm that had been treated with activin to be subjected to stationary culture in a physiological saline, more preferably in a physiological saline containing BSA (bovine fetal serum).

As a specific example of treatment with activin, a stationary culture treatment with activin for 0.5 to two hours at a concentration of 50 to 150 ng/ml, preferably at 80 to 120 ng/ml, can be given. Further, as a specific example of a method of treatment with retinoic acid, a stationary culture treatment with retinoic acid for 0.5 to two hours at a concentration of more than 10⁻⁵ M, preferably 10⁻⁴ M to 10⁻³ M can be given. Since retinoic acid is not water-soluble, it is preferable to use the acid by first dissolving with ethanol, dimethylsulfoxide (DMSO) and the like, then diluting with physiological saline. In addition, in the present invention, culture should be conducted after the treatment with activin and retinoic acid. As a specific example of culture after said treatment, a stationary culture in a physiological saline, preferably in a physiological saline containing BSA (bovine fetal serum) for five to 20 hours, preferably eight to 12 hours can be given.

There is no particular limitation to the pancreas induced in vitro in the present invention, as long as it can be obtained by the method of forming pancreas in vitro mentioned above. As referred to above, aside from the pancreas organ induced in vitro, an explant having an expression ability of a pancreas-specific molecular marker gene, an explant having a cytomorphology that is similar to pancreas in vivo, and an explant having a secretory gland-like structure that is similar to pancreas in vivo are also included. Furthermore, as to the screening method of the present invention, there is no particular limitation as long as it is a screening method of a substance that is useful for diagnosis, treatment and the like that use said pancreas obtained by the method of the present invention in vitro, such as a substance capable of curing hypofunction or dysfunction of pancreas, a substance capable of detecting hypofunction or dysfunction of pancreas, and the like. Screening of a substance that enhances or suppresses the function of pancreas can be conducted, for example, by injecting a test substance into pancreatic cell of the pancreas induced in vitro obtained from the present invention and comparing the expression ability of marker molecules such as insulin or the like to that of controls.

The present invention will be explained below in more detail with the examples, but the technical scope of the invention will not be limited to these examples.

### Example 1. (Preparation of presumptive ectoderm portion of a Xenopus late blastula)

The dorsal lymph sacs of adult male and female Xenopus laevis were each injected with 600 IU of hCG (human chorionic gonadotropin; Gestron; Denka Seiyaku, Japan), and fertilized eggs were obtained by mating these Xenopuses. These late blastulas [developmental stage 9] were dejellied in Steinberg's solution (SS: 58.00 mM NaCl 0.67 mM KCl, 0.34 mM Ca(NO₃)₂, 0.83 mM MgSO₄, 3.00 mM HEPES and 100 mg/l kanamycin sulfate; pH 7.4) containing 4.5% cysteine hydrochloride (pH 7.8), and the vitelline membranes were removed in the Steinberg's solution by using a pair of watchmaker's tweezers. The presumptive ectoderm portion of Xenopus late blastula thus obtained was cut into a size of 0.4 mm x 0.4 mm by using a tungsten needle.

### Example 2 (Tissues that differentiate by simultaneous treatment of the pieces of presumptive ectoderm with activin/retinoic acid for one hour)

Human recombinant activin A (Ajinomoto Co., Inc.) was dissolved in Steinberg's solution containing 0.1% bovine serum albumin (BSA) to a concentration of 100 ng/ml, and an activin solution was prepared. All-trans retinoic acid powder (CAT#R2625, Sigma) was first dissolved in ethanol to a concentration of 10⁻² M, and this ethanol solution, when treating the pieces of presumptive ectoderm, was diluted in the activin solution mentioned above to each of the concentration indicated in Table 1 to prepare each of the mixed solution of activin/retinoic acid, and were used in the experiment described below.

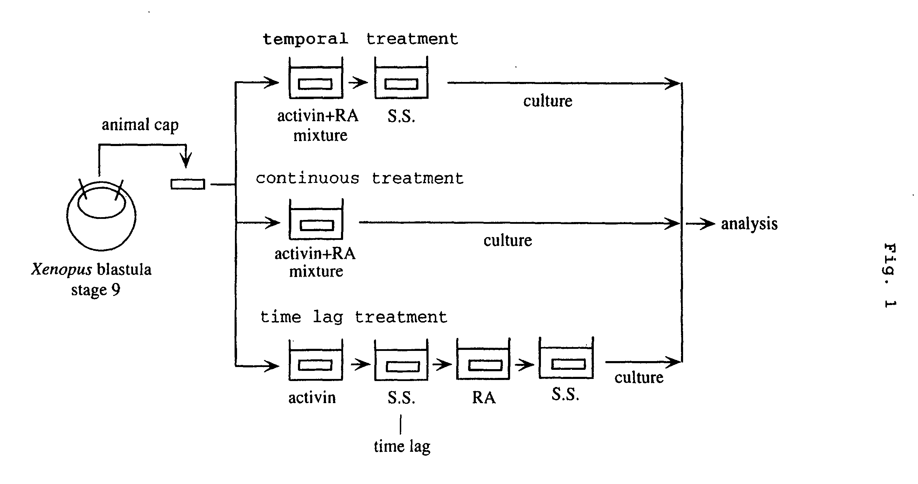

The pieces of presumptive ectoderm that had been cut out in Example 1 were treated by leaving at rest in a mixed solution of activin/retinoic acid prepared as mentioned above for one hour, washed two times with Steinberg's solution containing 0.1% BSA, and were cultured in the same solution for ten days at 20.degree. C. (see temporary treatment in Figure 1). These explants that had been cultured were fixed with Bouin' s solution, followed by dehydration treatment of a series of ethanol and xylene, and these explants were embedded in paraffin and sliced into 6 µm sections. These sections were stained with hematoxylin and eosin, and the tissues that differentiated were observed and examined under a light microscope. Further, the untreated pieces of presumptive ectoderm were also observed and examined in the same manner by using a light microscope. The results are shown in Table 1.

**Table 1.**

| | | | | | |
|---|---|---|---|---|---|
| Treatment time [h] | | | 1 | | |
| Concentration of activin [ng/ml] | | | 100 | | |
| Concentration of retinoic acid [M] | 0 | 10⁻⁷ | 10⁻⁶ | 10⁻⁵ | 10⁻⁴ |
| Number of samples | 31 | 29 | 33 | 30 | 31 |
| Atypical epidermis | 0 ( 0) | 0 ( 0) | 0 ( 0) | 0 ( 0) | 0 ( 0) |
| Epidermis | 30 ( 97) | 25 (86) | 28 ( 85) | 26 ( 87) | 25 (81) |
| Neural tissue | 12 ( 39) | 12 (41) | 15 (45) | 10(33) | 5 (16) |
| Notochord | 4 ( 13) | 2 ( 7) | 0 ( 0) | 0 ( 0) | 0 ( 0) |
| Muscle | 25 (81) | 26 ( 90) | 18 ( 55) | 6 ( 20) | 1 ( 3) |
| Pronephric tubule | 7 ( 23) | 6(21) | 11 (33) | 20 ( 67) | 19 (61) |
| Mesenchyme | 31 (100) | 26 ( 90) | 28 ( 85) | 24(80) | 23 ( 74) |
| Coelomic epithelium | 15 ( 48) | 16 ( 55) | 21(64) | 20 ( 67) | 23(74) |
| Cartilage | 5(16) | 7(24) | 3( 9) | 1 ( 3) | 0 ( 0) |
| Pharyngeal epithelium | 12(39) | 18(62) | 23(70) | 19(63) | 23(74) |
| Intestinal epithelium | 0 ( 0) | 0 ( 0) | 2 ( 6) | 5 ( 17) | 7 ( 23) |
| Pancreas | 0 ( 0) | 1 ( 3) | 0 ( 0) | 2 ( 7) | 5(16) |
| Yolk-rich cell | 18 ( 58) | 20 ( 69) | 24 ( 73) | 16(53) | 14 ( 45) |

| | | | | | |
|---|---|---|---|---|---|
| Numbers in parenthesis indicate the percentage. | | | | | |

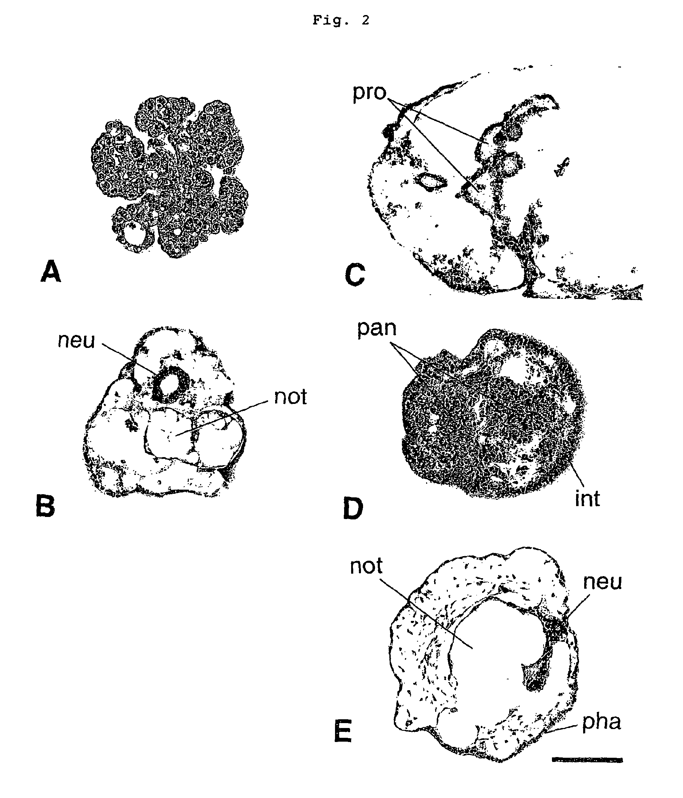

The results shown above revealed that the untreated pieces of presumptive ectoderm formed atypical epidermis four days after the beginning of culture (see Figure 2A). Further, when the pieces of presumptive ectoderm were treated only with activin (100 ng/ml), dorsal mesodermal and anterior endodermal tissues such as notochord, muscle, pharyngeal epithelium and the like differentiated. Neural tissues, which are presumed to have been induced secondarily by the dorsal mesoderm, were also partly detected (see Table 1 and Figure 2B). In the treatment with mixed solution of activin/retinoic acid, it was found out that as the concentration of retinoic acid increased, the formation rate of notochord, followed by the formation rate of muscle, decreased (Table 1) . Meanwhile, the formation rate of pronephric tubule increased, and reached the maximum rate when the concentration of retinoic acid was 10⁻⁵ to 10⁻⁴ M (more than 60%). Further, as the concentration of retinoic acid increased, the differentiation of intestinal epithelium was somewhat promoted (23% at 10⁻⁴ M). As to the formation of pancreas, differentiation was detected when the concentration of retinoic acid was high, but its ratio was low as 16% (10⁻⁴ M). As just described, when retinoic acid was added to activin, the differential pattern of mesodermal tissues changed greatly due to the effect of the concentration of retinoic acid, but hardly any changes were found in the differential pattern of endodermal tissues. Note that in Figure 2B, "not" represents notochord and "neu" represents neural tissue, respectively. The scale bar represents 100 µm.

### Reference Example 1 (Tissues that differentiate by simultaneous and continuous treatment of the pieces of presumptive ectoderm with activin/retinoic acid)

Based on the results from Example 2, since the effect of retinoic acid treatment was found in the differential pattern of mesodermal tissues, it can be considered that retinoic acid acted in the period when the fate of the mesoderm is determined to each mesodermal tissue (notochord, muscle, pronephric tubule and the like, for example). In contrast, it had been reported that in normal development, each endodermal tissue is formed later compared to the formation of each mesodermal tissue (Nieuwkoop, P. D. and Faber, J.; (1956) Normal table of Xenopus laevis (Daudin). (Amsterdam: North-Holland Publishing Company). Consequently, it can be presumed that the determination of differentiation to each endodermal tissue occurs in a later period compared to the determination to each mesodermal tissue. However, this period cannot be exactly specified in vitro. Consequently, in order to eliminate the effect caused by treatment time and timing, a continuous treatment with a mixed solution of activin/retinoic acid was conducted as follows. The pieces of presumptive ectoderm that had been cut out in Example 1 were cultured in the mixed solution of activin/retinoic acid prepared as described above at 20.degree. C. for 10 days (see continuous treatment in Figure 1). These explants that had been cultured were stained in the same manner as in Example 2, and the tissues that differentiated were observed and examined by using a light microscope. The results are shown in Table 2.

**Table 2.**

| | | | | | | |
|---|---|---|---|---|---|---|
| Concentration of activin [ng/ml] | | | 100 | | | |
| Concentration of retinoic acid [M] | 0 | 10⁻⁹ | 10⁻⁸ | 10⁻⁷ | 10⁻⁶ | 10⁻⁵ |
| Number of samples | 10 | 11 | 10 | 12 | 11 | 10 |
| Atypical epidermis | 0 ( 0) | 0 ( 0) | 0 ( 0) | 0 ( 0) | 0 ( 0) | 0 ( 0) |
| Epidermis | 9 (90) | 11 (100) | 10 (100) | 10 ( 83) | 10 ( 91) | 5 (50) |
| Neural tissue | 10 (100) | 8 ( 73) | 3 (30) | 7 ( 58) | 7 ( 64) | 0 ( 0) |
| Notochord | 3 (30) | 3 ( 27) | 1 (10) | 2 ( 17) | 0 ( 0) | 0 ( 0) |
| Muscle | 10 (100) | 11 (100) | 9 (90) | 10(83) | 9 (82) | 2 ( 20) |
| Pronephric tubule | 0 ( 0) | 0 ( 0) | 0 ( 0) | 0 ( 0) | 7 ( 64) | 5 (50) |
| Mesenchyme | 9 (90) | 11 (100) | 10 (100) | 11 (92) | 11 (100) | 9 (90) |
| Coelomic epithelium | 4(40) | 2(18) | 3(30) | 5(42) | 8 ( 73) | 7(70) |
| Cartilage | 1(10) | 1 ( 9) | 0 ( 0) | 0 ( 0) | 0 ( 0) | 0 ( 0) |
| Pharyngeal epithelium | 5(50) | 7 ( 64) | 8(80) | 9 ( 75) | 9(82) | 9(90) |
| Intestinal epithelium | 0 ( 0) | 0 ( 0) | 0 ( 0) | 0 ( 0) | 0 ( 0) | 2 ( 20) |
| Pancreas | 0 ( 0) | 0 ( 0) | 0 ( 0) | 0 ( 0) | 0 ( 0) | 0 ( 0) |
| Yolk-rich cell | 5(50) | 3 ( 27) | 4(40) | 7 ( 58) | 4(36) | 9(90) |

| | | | | | | |
|---|---|---|---|---|---|---|
| Numbers in parenthesis indicate the percentage. | | | | | | |

As can be seen from the results in Table 2, the formation rate of notochord and muscle decreased and the formation rate of pronephric tubule increased as the concentration of retinoic acid increased, in the same manner as when treated for one hour, also when treated continuously with the mixed solution of activin/retinoic acid (Table 2). In concentration of retinoic acid at 10⁻⁵ M, formation of pharyngeal epithelium was detected at a low rate (20%), but pancreas did not differentiate. Further, it was discovered that in treatment with retinoic acid at a concentration of 10-⁴ M, all the explants died within the culture period. Meanwhile, it has been reported by the present inventors that when a blastopore upper lip of an early gastrula is cut out and then treated with retinoic acid for one to three hours, the predeterimined fate of the cells are changed, and pancreas is formed (Moriya, N. et al.; Develop. Growth. Differ. 42, 175-185, 2000). In this case, at the time the treatment with retinoic acid begins, the blastopore upper lip is already directed to be a dorsal mesoderm/anterior endoderm. However, when the piece of presumptive ectoderm is treated in the manner as described above, it would be affected simultaneously by the action of high concentration activin (induction to dorsal mesoderm/anterior endoderm) and action of retinoic acid. In such a case, it is presumed that the presumptive ectodermal cells cannot become a dorsal mesoderm/anterior endoderm, and the lateral mesoderm is induced (Moriya, N. et al.; Develop. Growth. Differ. 35, 123-128, 1993). Furthermore, there is a possibility that the pancreas inductive action of retinoic acid is only effective to cells that have already been directed to dorsal mesoderm/anterior endoderm.

### Example 3 (Tissues that differentiate by treatment with time lag of the pieces of presumptive ectoderm with activin/retinoic acid)

A time lag was provided between the treatment with activin and treatment with retinoic acid, so that the action of retinoic acid can be received for the first time, after the differentiation to dorsal mesoderm/anterior endoderm had been determined by action of activin. The pieces of presumptive ectoderm that had been cut out in Example 1 were left at rest in 100 ng/ml activin solution for one hour, washed two times with Steinberg's solution containing 0.1% BSA, and were then left at rest in Steinberg's solution containing 0.1% BSA only for the period of each time lag indicated in Table 3. After the elapse of the time lag, the pieces of presumptive ectoderm were left at rest in retinoic acid solution at 10⁻⁴ M for one hour, washed two times with Steinberg's solution containing 0.1% BSA, and then cultured in Steinberg's solution containing 0.1% BSA at 20.degree. C. for ten days (see time lag treatment in Figure 1). These explants that had been cultured were stained in the same manner as described in Example 2, and the tissues that differentiated were observed and examined under a light microscope. The results are shown in Table 3.

**Table 3.**

| | | | | | | | | |
|---|---|---|---|---|---|---|---|---|
| Treatment time with activin [h] | | | | 1 | | | | 1^{b)} |
| Time lag [h] | - ^{a)} | 0 | 3 | | 5 | 15 | 25 | - |
| Treatment time with retinoic acid [h] | | | | 1 | | | | - |
| Number of samples | 59 | 65 | 31 | | 35 | 38 | 33 | 65 |
| Atypical epidermis | 0 ( 0) | 0 ( 0) | 0 ( 0) | | 0 ( 0) | 0 ( 0) | 0 ( 0) | 0 ( 0) |
| Epidermis | 45(76) | 48(74) | 13(42) | | 18(51) | 28(74) | 29(88) | 59(91) |
| Neural tissue | 5 ( 8) | 0 ( 0) | 0 ( 0) | | 0 ( 0) | 1 ( 3) | 1 ( 3) | 32 ( 49) |
| Notochord | 0 ( 0) | 0 ( 0) | 0 ( 0) | | 2 ( 6) | 5 ( 13) | 17(52) | 34(52) |
| Muscle | 3( 5) | 8(12) | 12(39) | | 8(23) | 23(61) | 16(48) | 37(57) |
| Pronephric tubule | 38 (64) | 39 (60) | 10(32) | | 4 ( 11) | 2 (5) | 0 (0) | 5 ( 8) |
| Mesenchyme | 47 (80) | 53 (82) | 27 ( 87) | | 30 ( 86) | 38 (100) | 32 ( 97) | 59 (91) |
| Coelomic epithelium | 40 ( 68) | 38 (58) | 22 (71) | | 15 (43) | 28 (74) | 19 (58) | 28 ( 43) |
| Cartilage | 0 ( 0) | 1 ( 2) | 0 ( 0) | | 0 ( 0) | 0 ( 0) | 0 ( 0) | 12 ( 18) |
| Pharyngeal epithelium | 50 ( 85) | 59 (91) | 30 ( 97) | | 27 ( 77) | 32 ( 84) | 29 ( 88) | 37 ( 57) |
| Intestinal epithelium | 11 ( 19) | 24 (37) | 19 (61) | | 29 ( 83) | 9 ( 24) | 3 ( 9) | 3 ( 5) |
| Pancreas | 20 ( 34) | 27 (42) | 25 (81) | | 29 ( 83) | 8(21) | 1 (3) | 0 ( 0) |
| Yolk-rich cell | 37 (63) | 37 ( 57) | 29 ( 94) | | 23 ( 66) | 18 (47) | 18 (55) | 29 ( 45) |

| | | | | | | | | |
|---|---|---|---|---|---|---|---|---|
| Numbers in parenthesis indicate the percentage. a) Simultaneous treatment with activin/retinoic acid b) Treatment with activin alone | | | | | | | | |

As can be seen from the results shown above, pronephric tubule differentiated with high efficiency when treated simultaneously with activin (100 ng/ml) and retinoic acid (10⁻⁴ M) or when treated with a time lag of 0 hour (treated with retinoic acid immediately after treatment with activin) (see Table 3 and Figure 2C). It was discovered that at time lag of three to five hours, the formation rate of pronephric tubule was low, and the formation rate of pancreas was highest at more than 80% (see Table 3, Figure 2D). It was found out that notochord and pharyngeal epithelium differentiate at time lag for more than 15 hours (see Table 3, Figure 2E). However, the formation rate of pancreas at a time lag of less than three hours or more than 15 hours was low (Table 3). Observation by light microscopy showed that two or more cells gathered to indicate a secretory gland (acinus)-like structure in pancreas at time lag of three to five hours, and cells having a clear lumen-like space in the center were found among these cell groups. Further, it was discovered that these pancreata were similar to pancreas of normal embryo, since the nucleus of these pancreata were positioned in the basal side of acinus, the vicinity of the center of the acinus were well-stained with eosin, and a racemose cell aggregate was formed (Figure 2D).

Based on these findings, the fact that differentiation into pancreas is occurred by providing a time lag suggests that it takes three to five hours for the presumptive ectodermal cells that have just been directed to dorsal mesoderm/anterior endoderm by activin to make all preparation for receiveing pancreas inductive action of retinoic acid effectively. It can be presumed that the action of retinoic acid before this period would affect the determination of mesodermal tissues that is about to occur, and the determination of endodermal tissues would be finished after this period. Therefore, it is considered that pronephric tubule was induced in the treatment with a time lag of one to three hours, pancreas was induced with a time lag of three to five hours, and notochord and pharyngeal epithelium were formed with a time lag of more than five hours in the same way as when untreated with retinoic acid. Note that in Figure 2C, Figure 2D and Figure 2E, "not" represents notochord, "neu" represents neural tissue, "pro" represents pronephric tubule, "pan" represents pancreas, "int" represents intestinal epithelium and "pha" represents pharyngeal epithelium, respectively. The scale bar represents 100 µm.

Furthermore, observation by light microscopy revealed that the pancreas that differentiated in the explants mentioned above, are surrounded by intestinal epithelium that has thickened. The endodermal epithelium continues from the mouth to the anus in a normal embryo, and its morphology changes continuously. Pharynx and intestine are both endodermal epithelium, however, it is known that the height of the cells are low and a morphology where cuboidal cells are lined is shown in the pharyngeal epithelium, while the height of the cells are tall and the epithelium is composed of tall cells lined in the intestinal epithelium (Chalmers, A. D. and Slack, J. M. W.; Dev. Dyn. 212, 509-521, 1998). Based on the thickness of the epithelium as a criterion, those with the "height/width" ratio of cells lower than three were classified as "pharyngeal epithelium" and those with the ratio higher than three were counted as "intestinal epithelium". It has been discovered that retinoic acid suppresses the formation of pharyngeal epithelium that is induced by treatment with activin, and induces pancreas and intestine, when a time lag of five hours is provided between the treatment with activin and the treatment with retinoic acid. It is known that in normal embryo and adult body of Xenopus, the pharynx is positioned in the anterior of the embryo, the pancreas and duodenum exist in the rear of the pharynx in the vicinity to each other, and the pancreas is connected to the duodenum to secrete digestive enzyme.

It has been known from the previous reports that Xenopus early embryos become head-deficient embryos when treated with retinoic acid (Durston, A. J. et al.; Nature 340, 140-144, 1989), that retinoic acid suppresses the anterior molecular marker and induces the posterior marker (Ruizi Altaba, A. and Jessell, T.; Development 112, 945-958, 1991, Ruizi Altaba, A. and Jessell, T.; Genes. Dev. 5, 175-187, 1991, Lopez, S. L. and Carrasco, A. E.; Mech. Dev. 36, 153-164, 1992, Kolm, P. J. et al.; Dev. Biol. 192, 1-6, 1997), and that retinoic acid and its receptor are localized in the posterior portion of embryo (Ellinger-Ziegelbauer, H. and Dreyer, C.; Genes. Dev. 5, 94-104, 1991, Chen, Y. et al.; Dev. Biol. 161, 70-76, 1994). Based on these findings, it can be presumed that the anterior endoderm induced by high concentration of activin (which differentiates into pharynx if nothing is done thereafter) was posteriorized by retinoic acid, and pancreas and intestinal epithelium were formed. In the table, the formation rate of pharyngeal epithelium appears to be high also when treated with high concentration of retinoic acid, because the cases where only a few pharyngeal epitheliums are found in the explants have also been counted. In the actual observation of the tissues, great many more intestinal epithelium were found to be coexistent with pancreas and to differentiate than pharyngeal epithelium (Figure 2D).

Moreover, there has been a report regarding the effect of retinoic acid to the endoderm of Xenopus embryo (Zeynali, B. and Dixon, K. E.; Dev. Genes. Evol. 208, 318-326, 1998). Xenopus embryo was treated with retinoic acid and the formation of gastrointestinal tract thereafter was examined, and an abnormality was found in the morphology of the gastrointestinal tract, but the formation of pancreas was found to be normal. It can be considered that the reason the retinoic acid did not affect the formation of pancreas here, is because the period the treatment was conducted was as late as developmental stage 22 to 32, and the effect to the whole embryo was considered. The present inventors conducted retinoic acid treatment to the whole embryos from the period of blastula to gastrula. These embryos became head-deficient embryos, however, deficiency and hypertrophy that are specific to endodermal organs were not found, and pancreas was not induced specifically in vivo. However, the physical relationship of each of the endodermal organs was abnormal and the endodermal organs were formed aggregately in the anterior and posterior axis direction. Based on these facts, it can be considered that retinoic acid does not have action to induce pancreas specifically, but induced differentiation of pancreas by the action to posteriorize the endodermal cells.

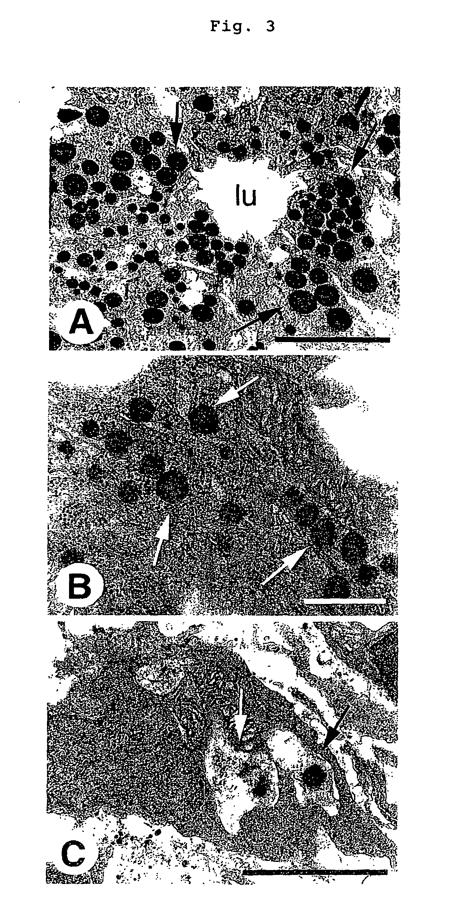

Secondarily, the pieces of presumptive ectoderm that had been cut out in Example 1 were treated in the same manner as described above, with the time lag of five hours, and the explant that had been cultured for 10 days at 20.degree. C. was pre-fixed in buffer I (3% paraformaldehyde, 2.5% glutaraldehyde, 0.1 M cacodylate; pH 7.4) for one day. This explant that had been fixed was washed with buffer I, fixed with buffer II (1% OsO₄, 0.1 M cacodylate; pH 7.4) for two hours, washed with buffer II, then dehydrated in a series of ethanol and acetone and embedded in epoxy resin. This explant that had been embedded was cut into thin slices and double-stained with uranyl acetate and lead citrate, then observed under a transmission electron microscope (JEM-200CX; JOEL) (see Figure 3). As a result, an exocrine gland-like structure wherein several cells are gathered was found in the explants. In the center of these cell groups, a space was found which looked like a lumen (Figure 3A), and in the opposite side of the space, that is, in the basal side of the acinous cell, nucleus was found, and many electron-dense secretory granule (0.2 to 1.0 µm in diameter) existed in the cells at the side of the space. It was revealed that these structures were very similar to the exocrine gland and exocrine granule of pancreas of normal embryos (Lozano, M. T. et al.; Gen. Comp. Endocrinol. 114, 191-205, 1999). In addition, cells that include two types of different secretory granules aside from the aforementioned were confirmed. One was a cell that includes electron-dense secretory granule (0.1 to 0.3 µm in diameter), which was similar to the glucagon producing cell of pancreas Langerhans' islet (Leone, F. et al.; L. Embryol. Exp. Morph. 36, 711-724, 1976, Lozano, M. T. et al.; Gen. Comp. Endocrinol. 114, 191-205, 1999) (Figure 3B). The other one was found to be a cell that has an electron-dense nucleus in the secretory granule (0.2 to 1.0 µm in diameter) and was similar to insulin producing cell (Leone, F. et al.; L. Embryol. Exp. Morph. 36, 711-724, 1976, Lozano, M. T. et al.; Gen. Comp. Endocrinol. 114, 191-205, 1999) (Figure 3C). The scale bars in Figure 3A, Figure 3B and Figure 3C represent 5, 1 and 1 µm, respectively. "lu" represents lumen, the arrow in Figure 3A represents exocrine granule, the arrow in Figure 3B represents the glucagon producing cell-like secretory granule, and the arrow in Figure 3C represents the insulin producing cell-like secretory granule, respectively.

### Example 4 (Expression of pancreas-specific gene)

The pieces of presumptive ectoderm that had been cut out in Example 1 were treated in the same manner as in Example 3 with a time lag of five hours, cultured for three days at 20.degree. C., and the expression of insulin that is a pancreas-specific gene (Henry, G. L. et al.; Development 122, 1007-1015, 1996) and XlHbox8 (Lemaire, P. et al.; Development 125, 2371-2380, 1998) were examined by the method as previously described (Yokota, C. et al.; J. Biochem. 123, 339-346, 1998). A mRNA was extracted from the explant that had been cultured, and cDNA was synthesized by using a reverse transcriptase (GIBCO BRL). One µl of the cDNA (2 µg/µl) that had been obtained was subjected to PCR, and the expression patterns of insulin that is a pancreas-specific gene and XlHbox8 (homologous to PDX1) were examined. EF-1α (elongation factor 1α) was used as a loading control. The combination of primers of each gene in PCR reaction that were used is as follows: insulin [insulin-F: 5'-ATGGCTCTATGGATGCAGTG-3' (Seq. ID No. 1), insulin-R: 5'-AGAGAACATGTGCTGTGGCA-3' (Seq. ID No. 2)], XlHbox8 [XlHbox8-F: 5'-CCTACAGCAACCCCTTGGTA-3' (Seq. ID No. 3), XlHbox8-R: 5'-GGGCTCTTGTGTAGGCTGTC-3' (Seq. ID No. 4)], EF-1α[EF-1α-F: 5'-TTGCCACACTGCTCACATTGCTTGC-3' (Seq. ID No. 5), and EF-1α-R: 5'-ATCCTGCTGCCTTCTTTTCCACTGC-3' (Seq. ID No. 6)].

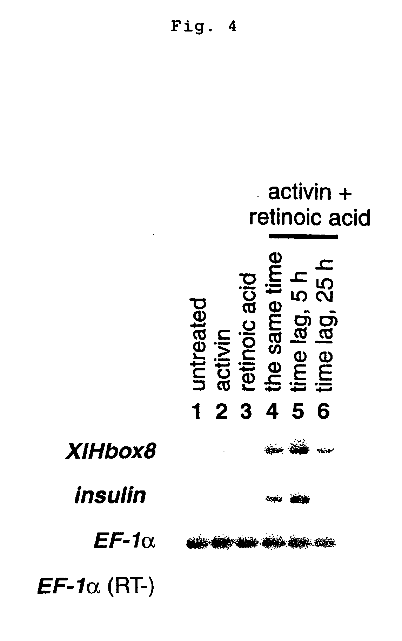

Seventy-six µl of DDW, 10 µl of 10 x Ex buffer, 8 µl of 2.5 mM dNTPs mix, 0.5 µl of 5U/pl ExTaq, and 0.5 µl of 100 pM of each of the above-mentioned primers were added to 5 µl of the above-mentioned cDNA (20 ng/µl), and PCR reaction was conducted with the total amount of 100 µl. The thermal cycle program, which is a cycle to denature for four minutes at 94.degree. C. only at the first time, followed by thermal denaturation for 30 seconds at 94.degree. C., stretched for one minute at 58.degree. C., and then annealing for one minute at 72.degree. C., was repeated for 23 to 30 times. Ultimately, annealing was conducted for nine minutes at 72.degree. C. After the PCR amplification product was isolated by agarose gel (1.5%) electrophoresis, it was detected by southern hybridization (Figure 4). As a negative control, EF-1α wherein RT-PCR was conducted under a condition where the reverse transcription factor had been eliminated was used.

It can be said from the result from Figure 4 described above that expression of these genes was not found in the untreated explants and in the explants treated with retinoic acid alone (lane 1 and 3 of Figure 4), and their expression was induced only a little in those treated with activin alone (lane 2 of Figure 4). However, it was revealed that the explants that had been simultaneously treated with activin and retinoic acid expressed these genes (lane 4 of Figure 4), and those that had been treated with activin and retinoic acid with a time lag of five hours showed higher expression (lane 5 of Figure 4). Moreover, as to those that had been treated with activin and retinoic acid with a time lag of 25 hours, their expression was lower compared to the case with a time lag of five hours (lane 6 of Figure 4). Based on these results, it was found out that there is small possibility that pancreas is differentiated specifically when treated with activin alone, and that pancreas is formed with high efficiency by being treated with activin and retinoic acid.

### Example 5 (Immunohistochemistry of explants treated with activin and retinoic acid)

The pieces of presumptive ectoderm that had been cut out in Example 1 were treated in the same manner as in Example 3 with a time lag of five hours, and were cultured for 10 days at 20. degree. C. The explant was fixed in buffer III [0.1 M 3-morpholinopropanesulfonic acid (MOPS), 2 mM EGTA, 1 mM MgSO₄, 3.7% formaldehyde], washed with physiological saline (PBS), followed by blocking with PBS containing 1% BSA, which contained 2% skimmed milk. This explant that had been blocked was left at rest for one night in a solution of an anti-insulin (guinea pig IgG) antibody (antibody dilution scale = 1:1000; CAT#A0564; Dako Corporation or antibody dilution scale = 1:1000; CAT#4010-01; Linco Research Inc.), or anti-glucagon (mouse IgG) antibody (antibody dilution scale = 1:2000; CAT#G-2654; Sigma), and then washed with PBS.

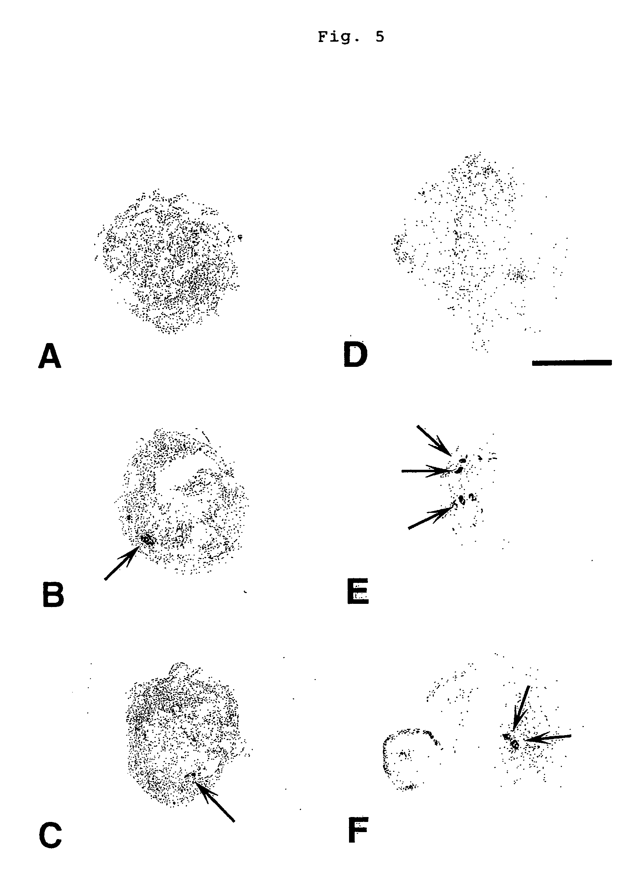

The explant that had been reacted with the primary antibody mentioned above and the explant which had not been reacted with the primary antibody used as a control were each reacted by using an anti-guinea pig IgG antibody labeled with alkaline phosphatase (antibody dilution scale = 1:500; CAT#61-4622; Zymed Laboratories Inc.) or an anti-mouse IgG antibody labeled with alkaline phosphatase (antibody dilution scale = 1:500; CAT#AQ160A; Chemicon International Inc.), then washed with PBS. The explants that had been reacted with these secondary antobodies were each colored blue in an alkaline phosphatase buffer [100 mM Tris-HCl, 5 mM MgCl₂, 100 mM NaCl, 0.1% Tween-20 (surface activating agent)] which includes nitrobenzoic acid (NIB) and 5-bromo-4-chloro-3-indolylphosphoric acid (BCIP) as a substrate. The explants were fixed again with Bouin's solution, then dehydrated in a series of ethanol and xylene. These explants were embedded in paraffin, cut into thin slices of 10 µm, and were observed under a transmission electron microscope (JEM-200CX; JOEL) (see Figure 5).

From the results shown in Figure 5 as described above, in controls reacted only with secondary antibody (anti-guinea pig IgG antibody labeled with alkaline phosphatase or anti-mouse IgG antibody labeled with alakaline phosphatase), none of the portions were stained in the explants with the use of any of them (Figure 5A, 5D) . However, when the primary antibody was an anti-insulin antibody, a few portions stained in the explants were confirmed (Figure 5B, 5C). Further, when the primary antibody was an anti-glucagon antibody, portions stained were confirmed as well (Figure 5E and 5F). Based on these results, it was found out that insulin and glucagon synthesize in the explants and that endocrine gland differentiates in the explants, by treatment with activin and retinoic acid with a time lag of five hours. Based on these findings, it can be considered that pancreas formed in vitro has a character similar to that of normal pancreas, not only morphologically but also functionally.

### Industrial Applicability

According to the present invention, a pancreas induced in vitro which enables to obtain knowledge regarding the mechanisms of differentiation and formation of pancreas that is useful for developmental engineering and organ engineering, a pancreas for transplantation by which it can be evaluated whether or not a pancreas induced in vitro can function in practice in vivo, and a pancreas induced in vitro which contributes to the development of diagnosis and treatment of pancreas disorder for higher animals, can artificially and efficiently be induced from a germ layer region apart from the presumptive region of pancreas. By the use of this method, it is possible to search for genes that are involved in the formation of pancreas, and analysis of the various genes for the formation of pancreas that had been obtained is possible, and using these genes enables to contribute to the development of gene therapy and gene diagnosis. Further, by transplanting these organs (pancreata) that had been formed in vitro, there is a possibility of the development of therapy for newborn that are born with insulin deficiency and for diseases involving genes. Still further, after grown to an adult, the activity of pancreas such as insulin is critical to lifestyle-related diseases such as diabetes and the like, and there is a possibility for development of therapies to the diseases caused by said diabetes and other functional changes of pancreas.

## Claims

1. A method of forming vertebrate pancreas in vitro, wherein a piece of presumptive ectoderm of a vertebrate blastula or gastrula is treated with activin and retinoic acid in vitro, then cultured; wherein treatment with activin is conducted, followed by treatment with retinoic acid after a predetermined time lag and wherein said vertebrate blastula or gastrula is non-human.

2. The method of forming vertebrate pancreas in vitro according to claim 1, wherein treatment with activin is conducted, followed by treatment with retinoic acid after three to 15 hours.

3. The method of forming vertebrate pancreas in vitro according claim 1 or claim 2, wherein the treatment with activin is a stationary culture treatment with activin at a concentration of 50 to 150 ng/ml for 0.5 to two hours.

4. The method of forming vertebrate pancreas in vitro according to any of claims 1 to 3, wherein the treatment with retinoic acid is a stationary culture treatment with retinoic acid at a concentration of more than 10⁻⁵ M for 0.5 to two hours.

5. The method of forming vertebrate pancreas in vitro according to any of claims 1 to 4, wherein the culture thereafter is a stationary culture treatment in a physiological saline.

6. The method of forming vertebrate pancreas in vitro according to any of claims 1 to 5, wherein the vertebrate is an animal that belongs to amphibian.

7. The method of forming vertebrate pancreas in vitro according to claim 6, wherein the animal that belongs to amphibian is a Xenopus.

8. A screening method for a substance capable of curing hypofunction or dysfunction of pancreas wherein said pancreas is obtained by the method of forming vertebrate pancreas in vitro according to any of claims 1 to 7.

## Patentansprüche

1. Verfahren zum Bilden von Wirbeltier-Pankreas in vitro, wobei ein Stück von mutmaßlichem Ektoderm einer Wirbeltier-Blastula oder -Gastrula in vitro mit Activin und Retinsäure behandelt und dann kultiviert wird, wobei die Behandlung mit Activin, gefolgt von der Behandlung mit Retinsäure nach einer vorbestimmten Zeitverzögerung durchgeführt wird und wobei die Wirbeltier-Blastula oder-Gastrula nicht menschlich ist.

2. Verfahren zum Bilden von Wirbeltier-Pankreas in vitro nach Anspruch 1, wobei die Behandlung mit Activin, gefolgt von der Behandlung mit Retinsäure nach drei bis 15 Stunden durchgeführt wird.

3. Verfahren zum Bilden von Wirbeltier-Pankreas in vitro nach Anspruch 1 oder Anspruch 2, wobei die Behandlung mit Activin eine stationäre Kulturbehandlung mit Activin in einer Konzentration von 50 bis 150 ng/ml für 0,5 bis zwei Stunden ist.

4. Verfahren zum Bilden von Wirbeltier-Pankreas in vitro nach einem der Ansprüche 1 bis 3, wobei die Behandlung mit Retinsäure eine stationäre Kulturbehandlung mit Retinsäure in einer Konzentration von mehr als 10⁻⁵ M für 0,5 bis zwei Stunden ist.

5. Verfahren zum Bilden von Wirbeltier-Pankreas in vitro nach einem der Ansprüche 1 bis 4, wobei die Kultur danach eine stationäre Kulturbehandlung in einer physiologischen Kochsalzlösung ist.

6. Verfahren zum Bilden von Wirbeitier-Pankreas in vitro nach einem der Ansprüche 1 bis 5, wobei das Wirbeltier ein Tier ist, welches zu den Amphibien gehört.

7. Verfahren zum Bilden von Wirbeltier-Pankreas in vitro nach Anspruch 6, wobei das zu den Amphibien gehörende Tier ein Xenopus ist.

8. Screening-Verfahren hinsichtlich einer Substanz, die in der Lage ist, eine Unterfunktion oder Fehlfunktion des Pankreas zu heilen, wobei der Pankreas durch das Verfahren zum Bilden von Wirbeltier-Pankreas in vitro nach einem der Ansprüche 1 bis 7 erhalten wird.

## Revendications

1. Procédé pour former un pancréas de vertébré in vitro, dans lequel un fragment d'ectoderme supposé d'une blastula ou gastrula de vertébré est traité avec de l'activine et de l'acide rétinoïque in vitro, puis cultivé ; dans lequel le traitement avec de l'activine est effectué, avec ensuite le traitement avec de l'acide rétinoïque après un intervalle de temps prédéterminé, ladite blastula ou gastrula de vertébré étant non humaine.

2. Procédé pour former un pancréas de vertébré in vitro suivant la revendication 1, dans lequel le traitement avec de l'activine est effectué, avec ensuite le traitement avec de l'acide rétinoïque après trois à 15 heures.

3. Procédé pour former un pancréas de vertébré in vitro suivant la revendication 1 ou la revendication 2, dans lequel le traitement avec de l'activine est un traitement en culture stationnaire avec de l'activine à une concentration de 50 à 150 ng/ml pendant 0,5 à deux heures.

4. Procédé pour former un pancréas de vertébré in vitro suivant l'une quelconque des revendications 1 à 3, dans lequel le traitement avec de l'acide rétinoïque est un traitement en culture stationnaire avec de l'acide rétinoïque à une concentration supérieure à 10⁻⁵ M pendant 0,5 à deux heures.

5. Procédé pour former un pancréas de vertébré in vitro suivant l'une quelconque des revendications 1 à 4, dans lequel la culture est ensuite un traitement en culture stationnaire dans du sérum physiologique.

6. Procédé pour former un pancréas de vertébré in vitro suivant l'une quelconque des revendications 1 à 5, dans lequel le vertébré est un animal qui fait partie des amphibiens.

7. Procédé pour former un pancréas de vertébré in vitro suivant la revendication 6, dans lequel l'animal qui appartient aux amphibiens est un Xenopus.

8. Procédé de sélection d'une substance capable de guérir un hypofonctionnement ou dysfonctionnement du pancréas, dans lequel ledit pancréas est obtenu par le procédé pour former un pancréas de vertébré in vitro suivant l'une quelconque des revendications 1 à 7.


### SEQUENCE LISTING

<110> JAPAN SCIENCE AND TECHNOLOGY CORPORATION
<120> Process for in vitro vertebrate pancreas formation
<130> A021-07PCT
<140>
   <141>
<150> 2000-161795
   <151> 2000-05-31
<160> 6
<170> Patent In Ver. 2.1
<210> 1
   <211> 20
   <212> DNA
   <213> Artificial Sequence
<220>
   <223> Description of Artificial Sequence:FRTM sequence
<400> 1
   atggctctat ggatgcagtg 20
<210> 2
   <211> 20
   <212> DNA
   <213> Artificial Sequence
<220>
   <223> Description of Artificial Sequence:Primer
<400> 2
   agagaacatg tgctgtggca 20
<210> 3
   <211> 20
   <212> DNA
   <213> Artificial Sequence
<220>
   <223> Description of Artificial Sequence:Primer
<400> 3
   cctacagcaa ccccttggta 20
<210> 4
   <211> 20
   <212> DNA
   <213> Artificial Sequence
<220>
   <223> Description of Artificial Sequence:Primer
<400> 4
   gggctcttgt gtaggctgtc 20
<210> 5
   <211> 25
   <212> DNA
   <213> Artificial Sequence
<220>
   <223> Description of Artificial Sequence:Primer
<400> 5
   ttgccacact gctcacattg cttgc 25
<210> 6
   <211> 25
   <212> DNA
   <213> Artificial Sequence
<220>
   <223> Description of Artificial Sequence:Primer
<400> 6
   atcctgctgc cttcttttcc actgc 25